# EUROPÄISCHE PATENTANMELDUNG

(11) **EP 2 014 264 A1**
(43) Veröffentlichungstag der Anmeldung: **14.01.2009**
(21) Anmeldenummer: 08158593.7
(22) Anmeldetag: 19.06.2008
(51) Int. Cl.: A61F 2/82, A61M 31/00

(54) **Implantat und System aus einem Implantat und einer Anregungsvorrichtung**

(30) Priorität: 13.07.2007 DE 102007032688
(71) Anmelder: Biotronik VI Patent AG, 6341 Baar (CH)
(72) Erfinder: Klocke, Björn, 8006, Zürich (CH); Fringes, Matthias, 91522, Ansbach (DE); Hofmann, Eugen, 8049, Zürich (CH)
(74) Vertreter: Lindner-Vogt, Karin L.

(57) **Zusammenfassung**

Die Erfindung betrifft ein Implantat mit einem Körper (1) und mindestens einer pharmazeutisch aktiven Substanz (4). Es wird vorgeschlagen, dass das Implantat ein relativ zu dem Körper auslenkbares Element (5,15,35,45,55) aufweist, das nach oder während einer äußeren Anregung der Auslenkung zur Freisetzung der mindestens einen pharmazeutisch aktiven Substanz (4) dient. Zudem wird ein System aus einem derartigen Implantat und einer Anregungsvorrichtung (30, 70) vorgeschlagen.

## Beschreibung

Die vorliegende Erfindung betrifft ein Implantat mit einem Körper und mindestens einer pharmazeutisch aktiven Substanz sowie ein System mit einem derartigen Implantat.

Im Zusammenhang mit der vorliegenden Erfindung werden als "Implantate" sämtliche lebensunfähige animale oder humane (xenogene oder allogene) oder künstliche Elemente bezeichnet, die in einen menschlichen oder tierischen Körper oder Organismus eingebracht werden können und die in dem jeweiligen Organismus zumindest über eine bestimmte Zeitdauer verbleiben. Zu derartigen Implantaten zählen beispielsweise Stents, Herzschrittmacher, künstliche Herzklappen, Knochen- oder Hautersatzstücke sowie Depot- oder Reservoirimplantate, die zur Freisetzung von Medikamenten über einen längeren Zeitraum dienen, z. B. Depotimplantate zur Behandlung von Krebserkrankungen wie beispielsweise Prostatakrebs, sowie biodegradierbare Gentamicin-Depotimplantate. Diese Implantate bestehen aus einem (Grund-)Körper oder Träger und ggf. weiteren Bestandteilen. Als ein derartiges weiteres Bestandteil werden heute vielfach pharmazeutisch aktive Substanzen wie Medikamente in oder an dem Körper vorgesehen, die über einen bestimmten Zeitraum im Organismus freigesetzt werden.

Eine "pharmazeutisch aktive Substanz" (oder therapeutisch aktive oder wirksame Substanz) im Sinne der Erfindung ist ein pflanzlicher, tierischer oder synthetisierter Wirkstoff (Medikament) oder ein Hormon, das in geeigneter Dosierung als Therapeutika zur Beeinflussung von Zuständen oder Funktionen des Körpers, als Ersatz für natürlich vom menschlichen oder tierischen Körper erzeugte Wirkstoffe, wie Insulin, sowie zur Beseitigung oder zum Unschädlichmachen von Krankheitserregern, Tumoren, Krebszellen oder Körperfremdstoffen Einsatz findet. Die Freisetzung der Substanz in der Endoprothesenumgebung hat einen positiven Effekt auf den Heilungsverlauf oder dient dem Unschädlich machen von maladen Zellen in der Onkologie bzw. wirkt pathologischen Veränderungen des Gewebes infolge eines chirurgischen Eingriffes entgegen.

Derartige pharmazeutisch aktive Substanzen weisen beispielsweise eine antiinflammatorische und/oder antiproliferative und/oder spasmolytische Wirkung auf, wodurch beispielsweise Restenosen, Entzündungen oder (Gefäß-)Spasmen vermieden werden können. Derartige Substanzen können in besonders bevorzugten Ausführungsbeispielen aus einer oder mehreren Substanzen der Wirkstoffgruppe der Calciumkanalblocker, der Lipidregulatoren (wie beispielsweise Fibrate), der Immunsuppressiva, der Calcineurininhibitoren (wie beispielsweise Tacrolimus), der Antiflogistika (wie beispielsweise Cortison oder Dichlofenac), der Antiinflammatorica (wie beispielsweise Imidazole), der Antiallergika, der Oligonucleotide (wie beispielsweise dODN), der Östrogene (wie beispielsweise Genistein), der Endothelbildner (wie beispielsweise Fibrin), der Steroide, der Proteine, der Hormone, der Insuline, der Zytostatika, der Peptide, der Vasodilatatoren (wie beispielsweise Sartane) und der antiproliferativ wirkenden Stoffe, bestehen.

Heute besteht vielfach der Wunsch, dass diese in einen menschlichen oder tierischen Körper eingebrachten Implantate die pharmazeutisch aktiven Substanzen nach Bedarf zu bestimmten Zeitpunkten und/oder über einen gewünschten Zeitraum, beispielsweise abhängig von dem Wert einer bestimmten Körperfunktion, z. B. dem Blutdruck; und/oder in einer gewünschten Menge in den Organismus, beispielsweise in das umgebende Gewebe oder die umgebende Körperflüssigkeit wie Blut freisetzen. Diese Freisetzung soll dabei beispielsweise durch den Arzt oder den Patienten mittels eines externen Geräts gesteuert werden können oder automatisch, beispielsweise abhängig von den Messwerten einer Sensorik für Körperfunktionen getriggert ausgegeben werden können. Durch eine derartige Freigabe der pharmazeutisch aktiven Substanz könnten Nebenwirkungen für den Patienten vermieden werden, da die Abgabe der pharmazeutisch aktiven Substanz lediglich in einem gewünschten Zeitfenster erfolgt. Es kann demnach durch eine solche Freigabe zu keiner Über- oder Unterdosierung kommen.

Ein besonders interessantes Anwendungsgebiet ist die Behandlung von Patienten mittels Stents. Stents sind endovaskuläre Prothesen, die zur Behandlung von Stenosen (Gefäßverengungen) eingesetzt werden können. Sie weisen ein rohrförmiges oder hohlzylinderförmiges Grundgitter als Körper auf, vorzugsweise bestehend aus zick-zack- oder mäanderförmig gefalteten, im Wesentlichen in Umfangsrichtung verlaufenden Stegen als Stützelemente und weiterhin bestehend aus diese Stützelemente verbindende, in Längsrichtung verlaufenden Stegen als Verbindungsstreben. Das Grundgitter ist an beiden Enden in Richtung der Achse des Hohlzylinders offen. Das rohrförmige Grundgitter einer derartigen Endoprothese wird in das zu behandelnde Gefäß eingesetzt und dient dazu, das Gefäß zu stützen. Derartige Stents haben sich insbesondere zur Behandlung von Gefäßerkrankungen etabliert. Durch den Einsatz von Stents können verengte Bereiche in den Gefäßen erweitert werden, so dass ein Lumengewinn resultiert. Nach dem Einsatz eines Stents besteht die Gefahr einer Restenose, d. h. einem Wiederverschließen des mit dem Stent behandelten Gefäßbereichs aufgrund verschiedener Prozesse, beispielsweise einer Koagulation der Körperflüssigkeit in diesem Bereich infolge einer Strömungsveränderung oder infolge eines Infektionsprozesses. Um Restenosen zu vermeiden, werden derartige Stents häufig mit pharmazeutisch aktiven Substanzen versehen, die beispielsweise eine antikoagulierende Wirkung besitzen. Auch im Hinblick auf einen derartigen Stent ist es wünschenswert, dass diese pharmazeutisch aktiven Substanzen gesteuert freigegeben werden.

Es sind bereits Stents mit Medikamentenabgabe aus einer Beschichtung bekannt, die beispielsweise aus einem Polymer bestehen kann. Außerdem werden bereits Medikamentendepots mit Pumpen verwendet. Nachteilig an den bekannten Lösungen ist jedoch, dass die pharmazeutisch aktiven Substanzen permanent abgegeben werden und keine Regelung der Freisetzung möglich ist. Zudem ist lediglich eine konstante Dosierung der Substanzen realisierbar und der Restbestand des Medikaments ist im Reservoir oder Depot nicht feststellbar. Der Organismus des behandelten Menschen oder Tieres durch die Dauerabgabe der Inhaltsstoffe belastet.

In der Druckschrift WO 2004/093643 A2 wird eine implantierbare Vorrichtung beschrieben, bei der eine therapeutische Substanz mittels eines magnetisierbaren Implantats an eine zu behandelnde Stelle befördert wird. Hierfür weist ein Implantat, das beispielsweise als Stent realisiert ist, eine Vielzahl von magnetisierbaren Eigenschaften auf, d. h. beispielsweise die Eigenschaft, permanentmagnetisch zu sein. Aufgrund der magnetisierbaren Eigenschaften des Implantats wird die therapeutische Substanz angezogen, wobei die therapeutische Substanz in einem magnetischen Träger angeordnet ist. Der magnetische Träger wird durch die Anwendung eines langreichweitigen magnetischen Feldes zu der Vorrichtung transportiert. Die bekannte Lösung dient somit dazu, eine nachträglich in den Organismus verabreichte therapeutische Substanz an einen bestimmten, vorgegebenen Ort zu transportieren. Das Problem, auch zeitlich die Abgabe einer pharmazeutisch aktiven Substanz zu steuern, wird hierdurch lediglich ungenügend gelöst, da bei dem bekannten Verfahren ein Transport der therapeutischen Substanz von dem Einbringungsort durch den Organismus zu dem Ort des Implantats erfolgt. Dieses ermöglicht keine exakte Voraussage darüber, zu welchem Zeitpunkt und in welcher Menge die gewünschte Konzentration der therapeutischen Substanz vorliegt. Zudem sind die Nebenwirkungen, die aus dem Transport der therapeutischen Substanz durch den Organismus resultieren können, nach wie vor vorhanden. Außerdem ist es häufig schwierig und kostenaufwendig, die therapeutischen Substanzen in einem geeigneten magnetischen Träger anzuordnen.

Aus der Druckschrift US 7,223,282 B1 ist eine implantierbare Vorrichtung in Form eines Stents bekannt, die ein erstes und ein zweites Material aufweist. Das erste Material weist eine therapeutische Substanz auf. Das zweite Material, das von dem Stent getragen wird, dient zur Umwandlung eines ersten Energietyps in thermische Energie, mittels der die therapeutische Substanz aus dem ersten Material freigesetzt wird. Die erzeugte thermische Energie lässt sich allerdings nur recht ungenau steuern, vor allem in Hinblick auf die freigegebene Menge, und beschädigt unter Umständen, wenn zu hohe Temperaturen erreicht werden, das die implantierbare Vorrichtung umgebende Gewebe des behandelten Patienten.

Die Aufgabe besteht somit darin, ein Implantat zu schaffen, mit welchem eine genaue zeitliche (d. h. im Hinblick auf Zeitpunkt und/oder Zeitdauer) und/oder genaue mengenmäßige Steuerung der Freigabe einer pharmazeutisch aktiven Substanz möglich ist, die das Gewebe des Patienten nicht beschädigt. Die Aufgabe besteht weiter darin, ein System zu schaffen, mittels dem die gewünschte Steuerung der Freigabe erreicht werden kann.

Die Aufgabe wird gelöst durch ein Implantat, das ein relativ zu dem Körper auslenkbares Element aufweist, das nach oder während einer äußeren Anregung der Auslenkung zur Freisetzung der mindestens einen pharmazeutisch aktiven Substanz dient. Hierbei wird unter "Auslenkung" eine mechanische Verschiebung des gesamten auslenkbaren Elements oder eines Teilbereichs des auslenkbaren Elements relativ zu dem Körper des Implantats oder eines Teils des Körpers des Implantats verstanden. Hierbei kann einerseits der Körper des Implantats zumindest an einem Punkt oder entlang einer Linie (vorzugsweise einer Geraden) fest mit dem Körper des Implantats verbunden sein. Außerdem wird unter "Auslenkung" auch die Verschiebung eines Teilbereichs des auslenkbaren Elements verstanden, wenn sich das gesamte auslenkbare Element oder dessen Schwerpunkt relativ zu dem Körper des Implantats nicht bewegt (z. B. bei einer Dickenschwingung). Durch das äußere Signal kann von einem Arzt oder Patienten mittels eines externen Gerätes als Anregungsvorrichtung oder mittels eines zweiten Implantats (beispielsweise über eine Triggerung durch Zeit oder durch eine in dem externen Gerät oder dem zweiten Implantat vorhandene Sensorik) die mindestens eine pharmazeutisch aktive Substanz gezielt zu einem bestimmten Zeitpunkt und über einen gewünschten Zeitraum freigegeben werden. Hierbei kann beispielsweise durch die steuerbare Amplitude oder Frequenz der Auslenkung des auslenkbaren Elements auch die Menge der abgegebenen pharmazeutisch aktiven Substanz gesteuert werden.

Hierdurch können Nebenwirkungen für den Patienten minimiert werden, da eine Dauerabgabe der pharmazeutisch aktiven Substanz nicht erfolgt. Zudem können Anwendungen der pharmazeutisch aktiven Substanz realisiert werden, die sonst nur durch die Gabe von Injektionen zu bewältigen sind, beispielsweise die Freigabe von Insulin, eine Hormonbehandlung (z. B. im Rahmen der Schwangerschaftsverhütung), bei der Behandlung von Suchtkranken, bei der ein Abklingen der Abgabe von pharmazeutisch aktiven Substanzen wünschenswert ist, bei der gezielten Tumorbehandlung in der Onkologie oder der gezielten Anregung mittels einer pharmazeutisch aktiven Substanz in der Orthopädie zur Knochenbildung. Diese Behandlungen lassen sich somit für den Patienten belästigungsfreier realisieren.

In einem bevorzugten Ausführungsbeispiel ist die Auslenkung des auslenkbaren Elements eine über eine vorgegebene Zeit andauernde Schwingung. Diese Auslenkung lässt sich besonders einfach realisieren. Hierbei sind verschiedene Schwingungen des auslenkbaren Elements denkbar. Im Fall der Ausbildung des auslenkbaren Elements als scheibenförmiges Element oder Plättchen könnte beispielsweise eine Dickenschwingung oder eine Längsschwingung realisiert werden. Bei einem scheibenförmigen auslenkbaren Element mit einer zylindrischen Form sind neben Dickenschwingungen auch Radialschwingungen denkbar. Bei einem quaderförmigen auslenkbaren Element können zudem Scherschwingungen realisiert werden. Im Falle der Realisierung des auslenkbaren Elements als hohlzylinderförmiges Element sind (Wand-)Dickenschwingungen, Radialschwingungen oder Längsschwingungen in Richtung der Längsachse denkbar.

Weiterhin bevorzugt ist, wenn die Frequenz des äußeren Anregungssignal so auf das auslenkbare Element abgestimmt ist, dass die Schwingung des Elements mit einer Resonanzfrequenz erfolgt. Alternativ kann die Geometrie und/oder Masse des Elements so auf die Frequenz des äußeren Anregungssignals abgestimmt sein, dass die Schwingung des Elements mit einer Resonanzfrequenz erfolgt. Die Steuerung der Abgabe ist hierdurch besonders einfach und gezielt möglich.

Eine Möglichkeit der Realisierung eines derartigen Implantats besteht darin, dass das auslenkbare Element mindestens teilweise ferromagnetisch oder permanentmagnetisch ausgebildet ist und die äußere Anregung mittels eines Signals eines magnetischen Wechselfelds erfolgt. Besonders bevorzugte Frequenzen der magnetischen Wechselfeder sind Frequenzen von etwa 100 Hz bis etwa 100 KHz. Beispielsweise können auslenkbare Elemente aus Kobalt oder Eisen verwendet werden. Hierbei ist es vorteilhaft, wenn der Körper des Implantats oder anderer Bestandteile des Implantats das magnetische Wechselfeld nicht abschwächen. Das Material des Implantats muss hierfür entsprechend gewählt werden, vorzugsweise weist das Material ein Element aus der Gruppe der paramagnetischen Metalle, welche eine positive magnetische Suszeptibilität besitzen (Alkalimetalle, Erdalkalimetalle oder Seltene Erden), auf. Allerdings wird durch den Wassergehalt des menschlichen oder tierischen Organismus', da Wasser diamagnetisch ist, ein äußeres magnetisches Anregungsfeld abgeschwächt und somit die Wirkstoffabgabe eingeschränkt. In diesem Fall wäre es denkbar, wenn das magnetische Anregungsfeld induktiv durch die Anwendung einer Spule in einem zweiten Implantat erzeugt wird. Hierbei dient das zweite Implantat als Anregungsvorrichtung und ist in der Nähe des ersten, die pharmazeutisch aktive Substanz abgebenden Implantats angeordnet.

Alternativ kann die äußere Anregung auch mittels eines Ultraschallsignals erfolgen, wobei das Ultraschallsignal so auf das auslenkbare Element und den Körper des Implantats abgestimmt ist, dass das auslenkbare Element relativ zu dem Körper des Implantats ausgelenkt wird. Hierbei kommen insbesondere Frequenzen im Bereich von 30 kHz bis 10 MHz in Frage. Welche Frequenz genau benötigt wird, ist jedoch vom Material und der Größe des Resonators (auslenkbares Element) abhängig. Die genau benötigte Frequenz f lässt sich nach der Formel f=c/R berechnen, wobei c die Schallgeschwindigkeit des jeweiligen Materials beschreibt (z.B. Stahl= 5000m/s, Wasser= 1500m/s) und R den Radius des Resonators. Die Anregung mittels Ultraschall hat den Vorteil, dass diese nicht durch Wasser abgeschwächt wird. Allerdings lassen sich durch Ultraschallsignale keine sehr tief liegenden Bereiche des zu behandelnden Organismus' erreichen.

Sowohl die Anregung durch ein magnetisches Wechselfeld als auch die Anregung mittels Ultraschall erlauben eine gezielte Freisetzung eines Medikaments, da die Medikamentenfreisetzung dann unterbrochen werden kann, wenn des Signal unterbrochen wird. Zudem kann auch die Signalamplitude zur Steuerung der Medikamentenabgabe dienen. Wird das Signal verstärkt, wird eine größere Menge der pharmazeutisch aktiven Substanz freigesetzt. Ein weiterer Vorteil, der durch die erfindungsgemäße Lösung erzielt werden kann, bestehen darin, dass sich auch über die Frequenz des magnetischen Wechselfelds bzw. des Ultraschalls die Dosierung, d. h. die Menge des freigesetzten pharmazeutisch aktiven Substanz einstellen lässt. Zudem ist es möglich, den Restbestand der pharmazeutisch aktiven Substanz in dem Implantat einfach durch Aufsummierung der gezielt veranlassten Freigaben zu bestimmen. Zudem ist es möglich, verschiedene pharmazeutisch aktive Substanzen (Wirkstoffe) auf oder in einem Implantat zu platzieren und ein oder mehrere dieser Substanzen gezielt, beispielsweise über eine für das auslenkbare Element des jeweiligen Wirkstoffs spezifische Anregungsfrequenz, welche eine entsprechend abgestimmte Resonanzfrequenz sein kann, anzuregen.

In einem bevorzugten, einfach und kostengünstig zu realisierenden Ausführungsbeispiel ist das Implantat derart ausgebildet, dass das auslenkbare Element plattenförmig gestaltet ist und vorzugsweise Öffnungen aufweist. Durch diese Öffnungen kann die mindestens eine pharmazeutisch aktive Substanz freigesetzt werden.

In einem weiteren Ausführungsbeispiel kann das auslenkbare Element auch als eine Schicht ausgebildet sein, die mit einer Vielzahl von auslenkbaren Elementen versehen ist. Diese auslenkbaren Elemente schwimmen in der Schicht, die mit der pharmazeutisch aktiven Substanz versehen ist. Jedes der Vielzahl von auslenkbaren Elementen ist vorzugsweise permanent- oder ferromagnetisch ausgebildet und demzufolge durch ein magnetisches Wechselfeld als Anregungssignal ansteuerbar. Bevorzugt ist die Folie biodegradierbar ausgebildet, so dass diese nach Verbrauch der mindestens einen pharmazeutisch aktiven Substanz abgebaut werden kann. Auch das gesamte Implantat kann vorzugsweise biodegradierbar ausgebildet sein.

Unter Biodegradation werden hydrolytische, enzymatische und andere stoffwechselbedingte Abbauprozesse im lebenden Organismus verstanden, die vor allem durch die mit der Folie oder dem Implantat in Kontakt gelangenden Körperflüssigkeiten verursacht werden und zu einer allmählichen Auflösung zumindest großer Teile der Folie oder des Implantats führen. Synonym zu dem Begriff Biodegradation wird häufig der Begriff Biokorrosion verwendet. Der Begriff Bioresorption umfasst die anschließende Resorption der Abbauprodukte durch den lebenden Organismus.

Für biodegradierbare Folien oder Implantate geeignete Werkstoffe können beispielsweise polymerer oder metallischer Natur sein. Das Implantat oder die Folie kann auch aus mehreren Werkstoffen bestehen. Gemeinsames Merkmal dieser Werkstoffe ist ihre Biodegradierbarkeit. Beispiele für geeignete polymere Verbindungen sind Polymere aus der Gruppe Cellulose, Kollagen, Albumin, Casein, Polysaccharide (PSAC), Polylactid (PLA), Poly-L-Lactid (PLLA), Polyglykol (PGA), Poly-D,L-Lactid-co-glycolid (PDLLA-PGA), Polyhydroxybuttersäure (PHB), Polyhydroxyvaleriansäure (PHV), Polyalkylcarbonate, Polyorthoester, Polyethylenterephtalat (PET), Polymalonsäure (PML), Polyanhydride, Polyphosphazene, Polyaminosäuren und deren Copolymere sowie Hyaluronsäure. Die Polymere können je nach den gewünschten Eigenschaften in Reinform, in derivatisierter Form, in Form von Blends oder als Copolymere vorliegen. Metallische biodegradierbare Werkstoffe basieren auf Legierungen von Magnesium, Eisen, Zink und/oder Wolfram.

In einem weiteren bevorzugten Ausführungsbeispiel ist das auslenkbare Element in einer Ausnehmung des Körpers des Implantats vorgesehen, wobei die mindestens eine pharmazeutisch aktive Substanz über oder unter dem Element ebenfalls in der Ausnehmung des Körpers des Implantats angeordnet ist. Hierdurch kann eine besonders einfache Freigabe der mindestens einen pharmazeutisch aktiven Substanz erfolgen. Bei einer überhalb des auslenkbaren Elements angeordneten pharmazeutisch aktiven Substanz bewirkt die Auslenkung des Elements, dass die pharmazeutisch aktive Substanz nach außen gedrückt wird. Bei der unterhalb des auslenkbaren Elements angeordneten Substanz wird diese durch die Auslenkung des Elements an die Oberfläche, beispielsweise durch Öffnungen in dem Element, befördert.

Besonders bevorzugt weist das auslenkbare Element durchgehende Öffnungen auf, die in dem Zustand ohne äußere Anregung verschlossen und in dem Zustand mit äußerer Anregung zumindest zeitweise offen sind und hierdurch die mindestens eine pharmazeutisch aktive Substanz freigeben. Das Öffnen und Schließen der durchgehenden Öffnungen kann beispielsweise durch eine Dickenschwingung des auslenkbaren Elements erreicht werden. In nicht angeregtem Zustand liegt das auslenkbare Element in Form eines Plättchens mit durchgehenden Öffnungen auf einem Bereich des Körpers des Implantats auf.

Hierdurch werden die Öffnungen des als Plättchen gestalteten auslenkbaren Elements verschlossen. Bei der Anregung vollführt das auslenkbare Element Dickenschwingungen, so dass das plättchenförmige auslenkbare Element von dem die Öffnungen verschließenden Bereich des Körpers des Implantats zeitweise abgehoben und hierdurch eine Freisetzung der mindestens einen pharmazeutisch aktiven Substanz bewirkt wird.

Eine weitere vorteilhafte Ausführungsform des erfindungsgemäßen Implantats besteht darin, dass das auslenkbare Element in einer durch den Körper des Implantats durchgehenden Öffnung angeordnet ist, die auf der einen Seite durch eine semipermeable Membran und auf der anderen Seite durch eine undurchlässige Kunststoffabdeckung verschlossen ist, wobei das auslenkbare Element und die mindestens eine pharmazeutisch aktive Substanz in der Öffnung zwischen der semipermeablen Membran und der Kunststoffabdeckung vorgesehen sind. Durch diese Ausgestaltung der Erfindung ist eine vor dem Einsetzen des Implantats durchzuführende einfache Befüllung der Öffnung mit der pharmazeutisch aktiven Substanz möglich.

In einem bevorzugten Ausführungsbeispiel hat die semipermeable Membran eine Porengröße von ca. 0,5 µm bis ca. 1 µm (abhängig von der Teilchengröße der verwendeten Substanz). Diese Porengröße ist deshalb vorteilhaft, da bei diesen Porengrößen einerseits wenig Diffusion auftritt und andererseits das Herausdrücken des Wirkstoffmoleküls ermöglicht wird.

Die obige Aufgabe wird außerdem durch ein System aus einem erfindungsgemäßen und oben beschriebenen ersten Implantat und einer Anregungsvorrichtung gelöst, wobei die Anregungsvorrichtung zur äußeren Anregung der Auslenkung des auslenkbaren Elements des ersten Implantats dient. Die Anregungsvorrichtung ist hierbei vorzugsweise als zweites Implantat ausgebildet, das in bevorzugter Weise in unmittelbarer Nähe, d. h. maximal in einer Entfernung zwischen etwa 5 cm und etwa 10 cm, des ersten Implantats angeordnet ist. Hierdurch kann die Anregung gezielter vorgenommen werden und das Anregungssignal wird weniger geschwächt, so dass die Freigabe der pharmazeutisch aktiven Substanz weniger fehlerbehaftet ist.

In einer bevorzugten Ausführungsform erzeugt die Anregungsvorrichtung ein magnetisches Wechselfeld oder ein Ultraschallsignal zur Anregung. Mittels dieser Anregungsformen ist eine besonders einfache, kostengünstige und besonders einfach zu steuernde Anregung möglich.

Um die Abschwächung des magnetischen Wechselfeldes durch das in dem behandelten Organismus eingelagerte Wasser zu vermeiden, erfolgt die Erzeugung des magnetischen Wechselfelds durch die Anregungsvorrichtung durch Anregung der Anregungsvorrichtung mit einer äußeren Induktionsspule.

In einem besonders bevorzugten Ausführungsbeispiel weist die Anregungsvorrichtung eine Sensoreinheit auf, mit der mindestens eine Körperfunktion gemessen wird, wobei die Anregungsvorrichtung derart gestaltet ist, dass die Abgabe eines Anregungssignals erst dann erfolgt, wenn mindestens eine der mindestens einen gemessenen Körperfunktion in einem vorgegebenen Bereich liegt. Die Freigabe der pharmazeutisch aktiven Substanz kann hierdurch noch gezielter erfolgen.

Alternativ zu der Möglichkeit, einer dauerhaften Anregung des auslenkbaren Elements z. B. bei der Resonanzfrequenz zu erzeugen, können auch Ultraschallimpulse oder magnetische Impulse mit einer hohen Amplitude, ggf. wiederholt, zur Anregung des auslenkbaren Elements genutzt werden.

Die Erfindung wird nachfolgend anhand von mittels Figuren dargestellten Ausführungsbeispielen näher erläutert. Dabei bilden alle beschriebenen und/oder bildlich dargestellten Merkmale den Gegenstand der Erfindung, unabhängig von ihrer Zusammenfassung in den Ansprüchen oder deren Rückbeziehung.

Es zeigen schematisch:
- Figur 1: Prinzipskizzen zu Schwingungstypen, die das auslenkbare Element eines erfindungsgemäßen Implantats je nach Gestaltung des auslenkbaren Elements ausführen kann,
- Figur 2: einen Querschnitt eines ersten Ausführungsbeispiels eines erfindungsgemäßen Implantats,
- Figur 3: einen Querschnitt eines zweiten Ausführungsbeispiels eines erfindungsgemäßen Implantats,
- Figur 4: einen Querschnitt eines dritten Ausführungsbeispiels eines erfindungsgemäßen Implantats,
- Figur 5: einen Querschnitt eines dritten Ausführungsbeispiels eines erfindungsgemäßen Implantats mit einer schematischen Darstellung einer Anregungsvorrichtung und
- Figur 6: eine Prinzipskizze für eine Anregungsvorrichtung.

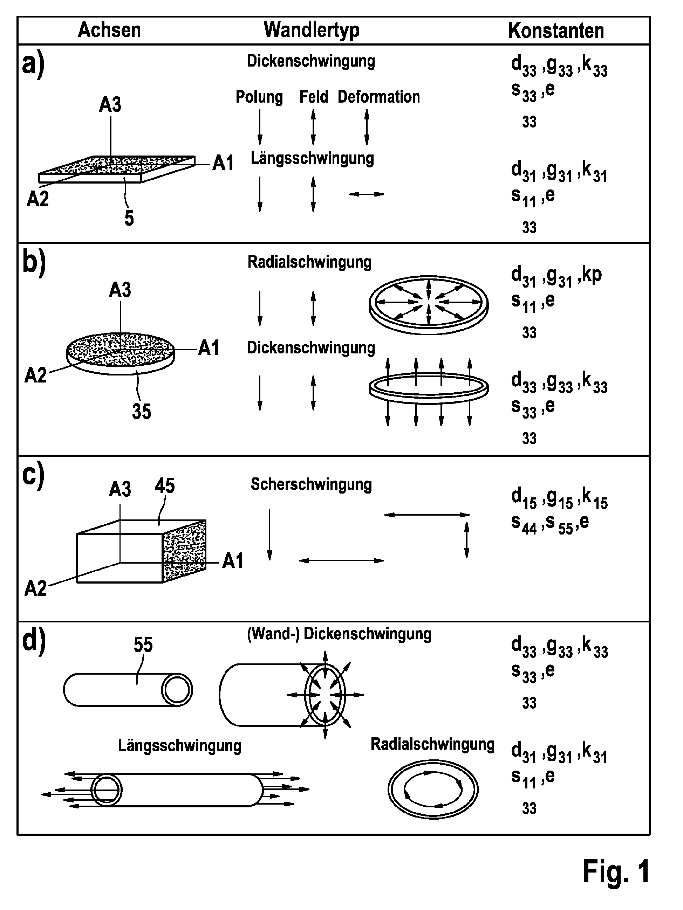

In Figur 1 sind verschiedene Realisierungsmöglichkeiten für ein auslenkbares Element eines erfindungsgemäßen Implantats dargestellt. Hierbei zeigt Figur 1a) ein plättchenförmiges auslenkbares Element 5 mit einer quadratischen Plättchenfläche. Eine rechteckige Plättchenfläche ist ebenfalls denkbar. Figur 1b) zeigt ein scheibenförmiges auslenkbares Element 35, das im wesentlichen als Kreisscheibe gestaltet ist, wobei die Ausgestaltung einer elliptischen Scheibe ebenfalls denkbar ist. Ein quaderförmiges auslenkbares Element 45 ist in Figur 1c) dargestellt. Figur 1d) zeigt ein hohlzylinderförmiges auslenkbares Element 55. Jeweils neben den auslenkbaren Elementen 5, 35, 45 und 55 sind in den Figuren 1a) bis 1d) die durch das Anregungssignal anregbaren Schwingungen mittels Pfeilen veranschaulicht. Das in Figur 1a) dargestellte plättchenförmige anregbare Element 5 kann demzufolge Dickenschwingungen und Längsschwingungen vollführen. Das in Figur 1b) dargestellte scheibenförmige auslenkbare Element 35 kann Radialschwingungen und Dickenschwingungen ausführen. Scherschwingungen sind bei dem quaderförmigen auslenkbaren Element 45 möglich. (Wand-)Dickenschwingungen, Radialschwingungen und Längsschwingungen können von dem hohlzylinderförmigen auslenkbaren Element 55 durchgeführt werden. Die x-, y-, und z-Achse der auslenkbaren Elemente 5, 35, 45 und 55 sind mit A1, A2 bzw. A3 bezeichnet.

Nachfolgend sollen Ausführungsbeispiele für ein erfindungsgemäßes Implantat anhand von Querschnitten eines Stents erläutert werden. Es versteht sich, dass der Aufbau der gezeigten Freigabeeinheit des jeweiligen Stents bestehend aus einem Element, in dem die pharmazeutisch aktive Substanz angeordnet ist, und dem auslenkbaren Element, auch in andere Implantate integriert werden kann.

Das erfindungsgemäße Implantat kann beispielsweise als Stent ausgebildet sein, in dessen Grundgitter als Körper des Stents, d. h. in oder auf dessen Stegen der Stützelemente oder Verbindungsstreben, eine oder mehrere pharmazeutisch aktive Substanzen angeordnet sind.

Der Körper eines derartigen erfindungsgemäßen Stents besteht vorzugsweise aus einem metallischen Material aus einem oder mehreren Metallen aus der Gruppe Eisen, Magnesium, Nickel, Wolfram, Titan, Zirkonium, Niob, Tantal, Zink und Silizium und ggf. einer zweiten Komponente aus einem oder mehreren Metallen aus der Gruppe Lithium, Natrium, Kalium, Kalzium und Mangan, vorzugsweise aus einer Zink-Kalziumlegierung. In einem weiteren Ausführungsbeispiel besteht der Grundkörper aus einem Formgedächtnis-Material aus einem oder mehreren Materialien aus der Gruppe bestehend aus Nickel-Titan-Legierungen und Kupfer-Zink-Aluminium-Legierungen, vorzugsweise aus Nitinol. In einem weiteren bevorzugten Ausführungsbeispiel besteht der Grundkörper des Stents aus Edelstahl, vorzugsweise aus einem Cr-Ni-Fe-Stahl - hier bevorzugt die Legierung 316L - oder einem Co-Cr-Stahl. Ferner kann die Grundgitter 2 mindestens teilweise aus Kunststoff und/oder einer Keramik bestehen.

In einem weiteren Ausführungsbeispiel besteht das Grundgitter aus einer absorbierbaren Magnesiumlegierung der folgenden Zusammensetzung:
- Seltene Erden 2,0 bis 30,0 Gew.%,
- Yttrium 2,0 bis 20,0 Gew.%,
- Zirkonium 0,3 bis 5 Gew.%,
- Rest 0 bis 10,0 Gew.% (ggf. z.B. Neodym),
wobei Magnesium (mindestens 60,0 Gew.%) den auf 100 Gew.% verbleibenden Gewichtsanteil an der Legierung einnimmt.

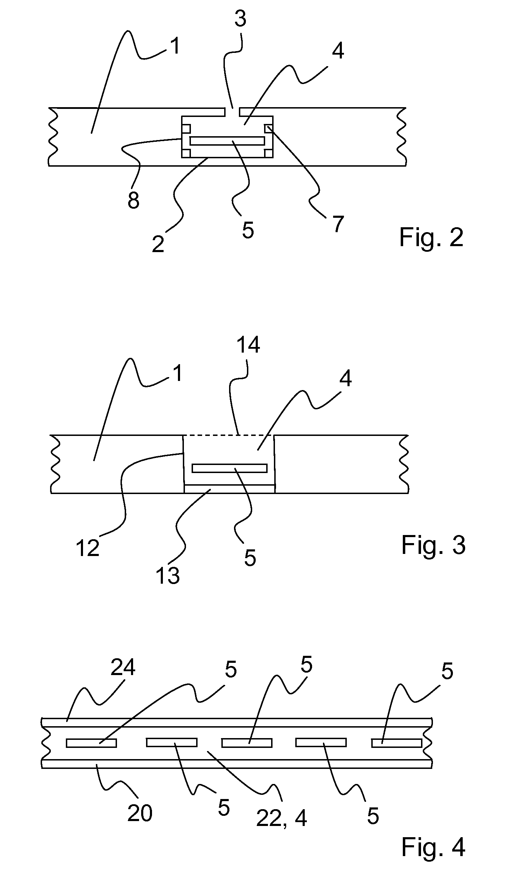

Figur 2 zeigt einen Querschnitt durch einen Körperabschnitt, d.h. eine Strebe 1, eines Stents. In der Strebe 1 ist eine Ausnehmung 2 angeordnet, die auf einer Seite der Strebe eine Öffnung 3 aufweist. In einem weiteren Ausführungsbeispiel können auch mehrere Öffnungen 3 vorgesehen sein. In der Ausnehmung 2 sind eine oder mehrere pharmazeutisch aktive Substanzen 4 angeordnet. Außerdem weist die Ausnehmung 2 ein plättchenförmiges auslenkbares Element 5 auf, das mit seinen zwei gegenüberliegenden Enden lose zwischen oberhalb und unterhalb des Elements 5 angeordneten, stufenförmigen Vorsprüngen 7 der Seitenwand 8 der Ausnehmung 2 angeordnet sind. Mittels einer äußeren Ultraschall-Anregung kann das plättchenförmige auslenkbare Element 5 in Schwingungen versetzt werden. Hierbei wird bevorzugt eine Eigenfrequenz des auslenkbaren Elements 5 angeregt, so dass dieses mit einer Resonanzfrequenz schwingt. Durch die Schwingung des auslenkbaren Elements 5, vorzugsweise in eine Richtung senkrecht zur Oberfläche des Plättchens 5, wird die in der Ausnehmung 2 darüber angeordnete pharmazeutisch aktive Substanz 4 durch die Öffnung 3 in den Organismus freigegeben, d. h. im Falle des Stents in die Körperflüssigkeit des Gefäßes, in dem der Stent angeordnet ist. Die Dicke der Stentstrebe 1 beträgt vorzugsweise etwa 1 mm.

In dem anhand von Figur 3 dargestellten Ausführungsbeispiel weist die Stentstrebe 1 eine durchgehende Öffnung 12 auf, die beispielsweise eine zylindrische Form hat. Diese Öffnung 12 ist auf der einen Seite durch eine undurchlässige Kappe 13 aus einem Polymermaterial verschlossen. Darüber ist in der durchgehenden Öffnung ein plättchenförmiges oder kreisscheibenförmiges auslenkbares Element 5 angeordnet. Oberhalb des plättchenförmigen auslenkbaren Elements 5 befindet sich eine oder mehrere pharmazeutisch aktive Substanz(en) 4 sowie, zum Verschließen der Öffnung 12 auf der der Kappe 13 gegenüber liegenden Seite, eine semipermeable Membran 14. Das plättchenförmige auslenkbare Element 5 kann entweder durch Ultraschall oder, wenn es mindestens teilweise ferromagnetisch oder permanentmagnetisch ausgebildet ist, mittels eines magnetischen Wechselfeldes angeregt werden. Hierdurch wird das plättchenförmige auslenkbare Element in Schwingungen versetzt und bewirkt die Freisetzung der pharmazeutisch aktiven Substanz(en) durch die semipermeable Membran 14 hindurch. Durch eine derartige Ausgestaltung können Stentstreben mit einer Dicke von etwa 100 µm mit einer oder mehreren pharmazeutisch aktiven Substanz(en) 4, die gezielt freigesetzt werden kann, ausgestattet werden. Außerdem ist eine einfache Befüllung der Öffnung 12 mit der(den) pharmazeutisch aktiven Substanz(en) 4 möglich.

In Weiterentwicklung der in Figur 3 dargestellten Ausführungsform kann die Wand eines Stentkörpers auch derart gestaltet werden, dass sie, wie in Figur 4 dargestellt, aus einer nicht permeablen oder permeablen Trägerschicht 20, einer dazwischen liegenden, mit einer oder mehreren pharmazeutisch aktiven Substanz(en) 4 versehenen Zwischenschicht 22 und einer permeablen Deckschicht 24 besteht. In der Zwischenschicht 22 sind eine Vielzahl von auslenkbaren Elementen 5 angeordnet, die ferromagnetisch oder permanentmagnetisch ausgebildet sind. Diese Elemente 5 sind vorzugsweise plättchenförmig ausgebildet und nebeneinander schwimmend in der flüssig oder gelartig ausgeführten Zwischenschicht 22 angeordnet. Wenn oberhalb oder unterhalb eines jeden Elements 5 eine pharmazeutisch aktive Substanz angeordnet ist, so kann diese während und nach einer Anregung (d. h. im Abklingen des Signals und/oder der Auslenkung) des entsprechenden Elements 5 durch die permeable Deckschicht 24 freigesetzt werden. Wenn die Trägerschicht 20 permeabel ausgeführt ist, so kann die Freisetzung auch durch diese Schicht hindurch erfolgen. Die nebeneinander angeordneten Elemente können z. B. in einzelnen, in der Zwischenschicht 22 vorhandenen Kammern mit verschiedenen pharmazeutisch aktiven Substanzen versehen sein, so dass diese jeweils spezifisch, bei entsprechender Bereitstellung des Anregungssignals bzw. entsprechender Ausgestaltung der Elemente 5, mit unterschiedlichen Anregungssignalen angeregt werden können, so dass die zugehörige pharmazeutisch aktive Substanz spezifisch freigesetzt wird.

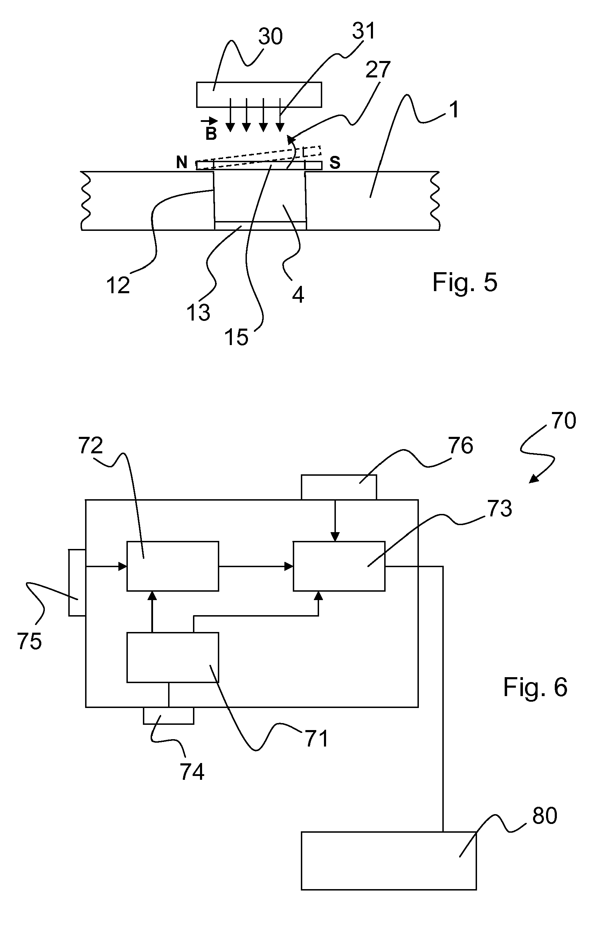

Das in Figur 5 dargestellte Ausführungsbeispiel eines erfindungsgemäßen Implantats weist eine auf der einen Seite mit einer Kappe 13 abgeschlossene durchgehende Öffnung 12 auf, die auf der anderen Seite mit einem plättchenförmigen auslenkbaren Element 15 abgedeckt ist. Dieses Element 15 ist magnetisiert. Die magnetischen Pole sind in Figur 5 mit den Buchstaben "N" und "S" gekennzeichnet. Wie Figur 5 zu entnehmen ist, ist die Magnetisierung so vorgesehen, dass je ein magnetischer Pole (N oder S) an je einem Ende des plättchenförmigen Elements 15 angeordnet ist. Hierbei ist einer dieser Pole (N oder S) an der Strebe beweglich (ähnlich eines Scharniers) befestigt (in dem dargestellten Ausführungsbeispiel Pol N). Bei einer Anregung mit einer Anregungsvorrichtung, die in dem in Figur 5 dargestellten Ausführungsbeispiel eine elektromagnetische Spule 30 aufweist, wird je nach Lage des durch die Spule 30 erzeugten Magnetfelds 31 (magnetischer Puls mit einer magnetischen Flussdichte B) entweder der magnetische Südpol S des ersten Endes des plättchenförmigen Elements 15 (siehe Fig. 5) oder in einem anderen, hier nicht dargestellten Ausführungsbeispiel, der magnetische Nordpol N des plättchenförmigen Elements 15 stärker durch die Spule 30 angezogen. Somit wird in dem in Figur 5 dargestellten Ausführungsbeispiel bei Anregung des Südpols S das magnetisierte Plättchen angehoben. Bei Anregung des magnetischen Nordpols N bleibt das Plättchen geschlossen. Die Bewegung des Elements 15 ist in Figur 5 durch den Pfeil 27 veranschaulicht. Das auslenkbare Element 15 wird hierdurch an einer Seite angehoben (siehe gestricheltes Plättchen). Durch diese Auslenkung des Elements 15 kann die in der Öffnung 12 angeordnete pharmazeutisch aktive Substanz 4 entweichen. Das anhand von Figur 5 geschilderte Prinzip entspricht dem Prinzip eines "Reed-Relais", bzw. des "Reed-Kontaktes". Nach Abschalten oder Umschalten (Umpolen) des Magnetfeldes der Spule 30 kehrt das plättchenförmige Element 15 wieder in seine Ausgangslage zurück. Diese Bewegung des Plättchens 15 kann mehrfach hintereinander wiederholt werden. Sowohl das plättchenförmige Element 15 als auch die Spule 30 (Magnet) sind in einem bevorzugten Ausführungsbeispiel nicht weit voneinander entfernt im Körper angeordnet. Der Abstand des plättchenförmigen Elements 15 und der Spule 30 soll vorzugsweise maximal etwa 5cm betragen. Die Spule 30 kann entweder ein Magnetfeld mit einer bestimmten, voreingestellten Frequenz erzeugen oder alternativ von außen steuerbar ausgebildet sein.

Die Auslenkungen der vorstehend beschriebenen auslenkbaren Elemente 5, 15, 35, 45, 55 betragen einige Nanometer bis einige Mikrometer.

Das in Figur 6 dargestellte Ausführungsbeispiel für eine erfindungsgemäße Anregungsvorrichtung 70 weist ein Netzteil 71 mit einem Gleichrichter, einen einstellbaren Frequenz-Sinusgenerator 72 und einen Verstärker/Endstufe 73 auf. Diese Elemente der Anregungsvorrichtung 70 sind elektrisch miteinander verbunden. Die Anregungsvorrichtung 70 weist außerdem einen Netzanschluss 74 auf, der mit dem Netzteil 41 verbunden ist. Mittels eines Bedienelements 45 zur Einstellung der Frequenz, das beispielsweise als Drehknopf ausgebildet ist, wird die zur Anregung des auslenkbaren Elements benötigte Frequenz eingestellt, die durch den Frequenz-Sinusgenerator 42 erzeugt wird. Die Amplitude von Strom und/oder Spannung kann durch das weitere Bedienelement 46 eingestellt werden, das vorzugsweise ein Bedienknopf oder mehrere Bedienknöpfe enthält. Das weitere Bedienelement 46 ist mit dem Verstärker/Endstufe 43 verbunden, wobei mittels des Verstärkers/Endstufe 43 ein Strom und/oder Spannung der vom Bediener eingestellten Amplitude erzeugt wird.

Der Verstärker/Endstufe 73 ist vorzugsweise über ein Kabel elektrisch mit der Sonde 80 verbunden, welche zur äußeren Anregung des jeweiligen auslenkbaren Elements dient und Bestandteil der Anregungsvorrichtung 70 ist. In dem in Figur 6 dargestellten Ausführungsbeispiel ist die Sonde 80 separat und bewegbar von den übrigen Bestandteilen der Anregungsvorrichtung 70 angeordnet.

Für die Anregung mittels eines magnetischen Wechselfelds weist die Sonde 80 vorzugsweise eine Spule aus Kupferdraht auf, die auf einem Weicheisenkern angeordnet ist. In einem weiteren Ausführungsbeispiel ist die Sonde 80 zusätzlich oder alternativ zur Anregung mittels magnetischem Wechselfeld zur Anregung mittels Ultraschall ausgebildet und enthält hierfür einen Piezokristall, der mit einem mechanischen Koppelelement verbunden ist, das sich bis zu mindestens einer Oberfläche der Sonde 80 erstreckt.

Für die Anregung des auslenkbaren Elements zur Freisetzung der pharmazeutisch aktiven Substanz wird die Sonde 80 im Falle der Anregung mittels eines magnetischen Wechselfelds nach dem Einstellen der Parameter Frequenz und Amplitude mittels der Bedienelemente 75 und 76 in einem kurzen Abstand (z.B. etwa 5 bis 10 cm) von dem beispielsweise auf einer Liege liegenden Patienten positioniert. Anschließend wird die Anregung des auslenkbaren Elements des in den Körper des Patienten implantierten Implantats mittels des magnetischen Wechselfelds vorgenommen.

Im Fall der Anregung mittels Ultraschall wird die Sonde 80 nach der Einstellung der Parameter an der Anregungsvorrichtung 70 auf den Körper des Patienten aufgesetzt. Vorab wurde an der Stelle, an der die Sonde 80 aufgesetzt werden soll, ein Gel aufgetragen, so dass das mechanische Koppelelement der Sonde 80 über das Gel mit dem Körper des Patienten verbunden ist. Anschließend wird das im Körper des Patienten implantierten Implantats durch die Anregungsvorrichtung 70 mittels Ultraschall angeregt, so dass die pharmazeutisch aktive Substanz durch Auslenkung des auslenkbaren Elements freigesetzt wird.

### Bezugszeichenliste:

- 1: Strebe eines Stentkörpers
- 2: Ausnehmung
- 3: Öffnung
- 4: pharmazeutisch aktive Substanz(en)
- 5: plättchenförmiges auslenkbares Element
- 7: Vorsprung an der Seitenwand 8
- 8: Seitenwand der Öffnung 3
- 12: durchgehende Öffnung
- 13: Kappe
- 14: semipermeable Membran
- 15: plättchenförmiges auslenkbares Element mit Magnetisierung
- 20: Trägerschicht
- 22: Zwischenschicht
- 24: Deckschicht
- 27: Pfeil
- 30: Spule
- 31: Magnetfeld
- 35: scheibenförmiges auslenkbares Element
- 45: quaderförmiges auslenkbares Element
- 55: hohlzylinderförmiges auslenkbares Element
- 70: Anregungsvorrichtung
- 71: Netzteil
- 72: Frequenz-Sinusgenerator
- 73: Verstärker/Endstufe
- 74: Netzanschluss
- 75: Bedienelement zu Einstellung der Frequenz
- 76: weiteres Bedienelement zur Einstellung der Amplitude von Strom und/oder Spannung
- 80: Sonde
- A1: x-Achse
- A2: y-Achse
- A3: z-Achse

## Patentansprüche

1. Implantat mit einem Körper und mindestens einer pharmazeutisch aktiven Substanz, **dadurch gekennzeichnet, dass** das Implantat ein relativ zu dem Körper (1) auslenkbares Element (5,15,35,45,55) aufweist, das nach oder während einer äußeren Anregung der Auslenkung zur Freisetzung der mindestens einen pharmazeutisch aktiven Substanz (4) dient.

2. Implantat nach Anspruch 1, **dadurch gekennzeichnet, dass** die Auslenkung eine über eine vorgegebene Zeit andauernde Schwingung ist.

3. Implantat nach Anspruch 2, **dadurch gekennzeichnet, dass** die Frequenz des äußeren Anregungssignals so auf das Element (5,15,35,45,55) abgestimmt ist, dass die Schwingung des Elements (5,15,35,45,55) mit einer Resonanzfrequenz erfolgt.

4. Implantat nach Anspruch 2, **dadurch gekennzeichnet, dass** die Geometrie und/oder Masse des Elements (5,15,35,45,55) so auf die Frequenz des äußeren Anregungssignals abgestimmt ist, dass die Schwingung des Elements (5,35,15,45,55) mit einer Resonanzfrequenz erfolgt.

5. Implantat nach einem der vorhergehenden Ansprüche, **dadurch gekennzeichnet, dass** das auslenkbare Element (5,15,35,45,55) mindestens teilweise ferromagnetisch oder permanentmagnetisch ausgebildet ist und die äußere Anregung mittels eines Signals eines magnetischen Wechselfeldes erfolgt.

6. Implantat nach einem der Ansprüche 1 bis 4, **dadurch gekennzeichnet, dass** die äußere Anregung mittels eines Ultraschallsignals erfolgt, wobei das Ultraschallsignal so auf das auslenkbare Element und den Körper (1) abgestimmt ist, dass das auslenkbare Element relativ zu dem Körper (1) ausgelenkt wird.

7. Implantat nach einem der vorhergehenden Ansprüche, **dadurch gekennzeichnet, dass** das auslenkbare Element (5) plattenförmig ausgebildet ist und vorzugsweise mindestens eine Öffnung aufweist.

8. Implantat nach einem der Ansprüche 1 bis 6, **dadurch gekennzeichnet, dass** eine Vielzahl von auslenkbaren Elementen (5) schwimmend in einer mit der mindestens einen pharmazeutisch aktiven Substanz versehenen, vorzugsweise degradierbaren Schicht (22) angeordnet ist, die vorzugsweise durch eine Folie gebildet ist.

9. Implantat nach einem der vorhergehenden Ansprüche, **dadurch gekennzeichnet, dass** das auslenkbare Element (5) in einer Ausnehmung (2,12) des Körpers (1) vorgesehen ist, wobei die mindestens eine pharmazeutisch aktive Substanz (4) über oder unter dem Element (5) angeordnet ist.

10. Implantat nach Anspruch 9, **dadurch gekennzeichnet, dass** das auslenkbare Element (5) durchgehende Öffnungen (12) aufweist, die in dem Zustand ohne äußere Anregung verschlossen und in dem Zustand mit äußerer Anregung zumindest zeitweise offen sind und hierdurch die mindestens eine pharmazeutisch aktive Substanz (4) freigeben.

11. Implantat nach einem der vorhergehenden Ansprüche, **dadurch gekennzeichnet, dass** das auslenkbare Element (5) in einer durch den Körper durchgehenden Öffnung (12) angeordnet ist, die auf der einen Seite durch eine semipermeable Membran (14) und auf der anderen Seite durch eine undurchlässige Kunststoffabdeckung (13) verschlossen ist, wobei das auslenkbare Element (5) und die mindestens eine pharmazeutisch aktive Substanz (4) in der Öffnung (12) zwischen der semipermeablen Membran (14) und der Kunststoffabdeckung (13) vorgesehen sind.

12. Implantat nach Anspruch 11, **dadurch gekennzeichnet, dass** die semipermeable Membran (14) eine Porengröße von ca. 0,5 µm bis ca. 1 µm aufweist.

13. System aus einem ersten Implantat nach einem der vorhergehenden Ansprüche und einer vorzugsweise als zweites Implantat ausgebildeten Anregungsvorrichtung (30, 70), wobei die Anregung zur äußeren Anregung der Auslenkung des auslenkbaren Elements (5) des ersten Implantats dient.

14. System nach Anspruch 13, **dadurch gekennzeichnet, dass** die Anregungsvorrichtung (30, 70) ein magnetisches Wechselfeld oder ein Ultraschallsignal zur Anregung erzeugt und dass die Erzeugung des magnetischen Wechselfelds durch die Anregungsvorrichtung mittels einer äußeren Induktionsspule erfolgt.

15. System nach Anspruch 13 oder 14, **dadurch gekennzeichnet, dass** die Anregungsvorrichtung eine Sensoreinheit aufweist, mit der mindestens eine Körperfunktion gemessen wird, wobei die Anregungsvorrichtung derart gestaltet ist, dass die Abgabe eines Anregungssignals erst dann erfolgt, wenn mindestens eine der mindestens einen gemessenen Körperfunktion in einem vorgegebenen Bereich liegt.
